# EUROPÄISCHE PATENTSCHRIFT

(11) **EP 3 073 935 B1**
(45) Veröffentlichungstag und Bekanntmachung des Hinweises auf die Patenterteilung: **25.03.2020**
(21) Anmeldenummer: 14805533.8
(22) Anmeldetag: 24.11.2014
(51) Int. Cl.: A61B 17/12

(54) **SYSTEM ZUM VERBINDEN EINES MEDIZINISCHEN IMPLANTATS MIT EINER EINFÜHRHILFE**
SYSTEM FOR CONNECTING A MEDICAL IMPLANT TO AN INSERTION AID
SYSTÈME SERVANT À RELIER UN IMPLANT MÉDICAL À UN AUXILIAIRE D'INSERTION

(30) Priorität: 29.11.2013 DE 202013105452 U
(43) Veröffentlichungstag der Anmeldung: 05.10.2016
(73) Patentinhaber: PFM Medical AG, 50996 Köln (DE)
(72) Erfinder: SCHÄFER, Joachim, 66620 Nonnweiler (DE)
(74) Vertreter: Hohendorf, Carsten
(86) Internationale Anmeldenummer: PCT/EP2014/075389
(87) Internationale Veröffentlichungsnummer: WO 2015/078807

(56) Entgegenhaltungen:
- EP-A1- 1 728 477
- WO-A1-96/40024
- WO-A2-2007/106495
- DE-A1-102007 038 446

## Beschreibung

Die Erfindung betrifft ein System zum Verbinden eines medizinischen Implantats mit einer Einführhilfe. Ein Implantat ist eine im menschlichen oder tierischen Körper eingepflanzte, künstliche Vorrichtung, welche permanent oder temporär innerhalb des menschlichen oder tierischen Körpers verbleibt.

Ein Beispiel für ein medizinisches Implantat ist ein sogenannter Stent, welcher in Hohlorgane des menschlichen oder tierischen Körpers eingebracht wird, um diese offen zu halten. Bei einem Stent kann es sich beispielsweise um ein kleines Gittergerüst in Röhrchenform aus Metall oder Kunstfasern handeln. Verwendung finden Stents zum einen in Blutgefäßen, speziell in den Herzkranzgefäßen, um nach deren Aufdehnung einen erneuten Verschluss zu verhindern. Zum anderen dienen Stents in der Krebsbehandlung dazu, durch bösartige Tumore verursachte Verengungen nach einer Aufdehnung offen zu halten.

Weiterhin finden medizinische Implantate in der Herzchirurgie Anwendung, wobei die medizinischen Implantate meist dazu dienen, eine abnormale Öffnung innerhalb des Herzens zu verschließen.

Soweit möglich werden derartige medizinische Implantate mittels eines minimalinvasiven Verfahrens in den menschlichen oder tierischen Körper eingebracht. Bei einem minimalinvasiven Verfahren wird ein Zugang zu einem großen Blutgefäß innerhalb des menschlichen oder tierischen Körpers geschaffen, über welchen ein Katheter in den menschlichen oder tierischen Körper eingeführt wird. Über die Blutgefäße wird dieser Katheter zur Implantationsstelle geführt. Nachfolgend wir über den Katheter das medizinische Implantat mittels einer Einführhilfe transportiert.

Üblicherweise bestehen die medizinischen Implantate aus einem Formgedächtnismaterial, so dass sich das medizinische Implantat nach Verlassen des Katheters in eine vorgegebene Form entfaltet. Das medizinische Implantat wird also mittels der Einführhilfe über den Katheter zu der Implantationsstelle transportiert und entfaltet sich nach Verlassen des Katheters in die vorgegebene Form. Nachdem das medizinische Implantat korrekt an der Implantationsstelle in den menschlichen oder tierischen Körper eingesetzt wurde, kann die Verbindung zwischen Einführhilfe und medizinischen Implantat gelöst werden.

Folglich muss das medizinische Implantat während des Transports durch den Katheter möglichst fest mit der Einführhilfe verbunden sein und sich gleichzeitig nach erfolgter Implantation einfach von der Einführhilfe lösen lassen. Aus dem Stand der Technik ist es beispielsweise bekannt, das medizinische Implantat auf einen Trägerdraht der Einführhilfe aufzuklemmen und die Klemmverbindung nach erfolgreicher Implantation des medizinischen Implantats zu lösen. Nachteilig an einer derartigen Verbindung zwischen dem medizinischen Implantat und der Einführhilfe ist jedoch, dass zum Lösen der Verbindung zwischen Implantat und Trägerdraht eine relativ hohe Kraft notwendig ist und weiterhin kann die Verbindung zum medizinischem Implantat und Trägerdraht, welcher auf einer Reibkraft basiert, beispielsweise durch Flüssigkeiten negativ beeinflusst werden, so dass sich die Verbindung zwischen medizinischen Implantat und Trägerdraht frühzeitig lösen kann.

Die WO 96/40024 A1 offenbart beispielsweise ein System zur Empfängnisverhütung umfassend ein als Primärspirale ausgebildetes Implantat und eine als Kerndraht ausgebildete Einführhilfe. Mittels des Kerndrahts kann das Implantat in einen Eileiter eingesetzt werden., wobei der Kerndraht das Implantat während der Implantation in einem gestreckten Zustand hält. Durch eine Drehbewegung relativ zueinander werden der Kerndraht und das Implantat miteinander verschraubt. Nach dem Platzieren des Implantats kann der Kerndraht erst nach dem Lösen der Schraubverbindung aus dem Implantat herausgezogen werden.

Der Erfindung liegt somit die Aufgabe zugrunde, ein System zum Verbinden, eines medizinischen Implantats mit einer Einführhilfe bereitzustellen, welches während der Implantation des medizinischen Implantats im menschlichen oder tierischen Körper eine sichere Verbindung zwischen medizinischen Implantat und Einführhilfe gewährleistet und gleichzeitig ein einfaches Lösen der Verbindung zwischen medizinischen Implantat und Einführhilfe nach erfolgter Implantation des medizinischen Implantats ermöglicht. Ferner sollte die Verbindung zwischen dem medizinischen Implantat und der Einführhilfe aufgrund eines Einsatzes in der minimalinvasiven Chirurgie einen möglichst geringen Durchmesser aufweisen, vorzugsweise einen Durchmesser der kleiner oder gleich dem Durchmesser des medizinischen Implantats bzw. der Einführhilfe ist.

Die Aufgabe wird erfindungsgemäß gelöst durch ein System zum Verbinden eines medizinischen Implantats mit einer Einführhilfe, umfassend eine erste spiralförmige Verbindungseinrichtung am proximalen Ende des medizinischen Implantats, eine zweite spiralförmige Verbindungseinrichtung am distalen Enden der Einführhilfe und einen Kerndraht, welcher in einem ersten Betriebszustand des Systems die erste spiralförmige Verbindungseinrichtung und die zweite spiralförmige Verbindungseinrichtung relativ zueinander fixiert, wobei in dem ersten Betriebszustand die erste spiralförmige Verbindungseinrichtung und die zweite spiralförmige Verbindungseinrichtung zumindest teilweise ineinandergreifen und sich der Kerndraht durch die erste und zweite spiralförmige Verbindungseinrichtung hindurch erstreckt.

Das erfindungsgemäße System ist somit derart ausgebildet, dass die Wendeln der ersten spiralförmigen Verbindungseinrichtung in die Wendeln der zweiten spiralförmigen Verbindungseinrichtung eingreifen und die erste spiralförmige Verbindungseinrichtung und die zweite spiralförmige Verbindungseinrichtung mittels des Kerndrahts relativ zueinander fixiert werden. Eingreifen im Sinne der Erfindung ist insbesondere ein seitliches Ineinanderschieben der spiralförmigen Verbindungseinrichtungen. Somit ist eine sichere Verbindung zwischen der Einführhilfe und dem medizinischen Implantat während des Transports des medizinischen Implantats zur Implantationsstelle, beispielsweise durch einen Katheter, gewährleistet. Nach erfolgreicher Implantation des medizinischen Implantats im menschlichen oder tierischen Körper kann die Verbindung zwischen dem medizinischen Implantat und der Einführhilfe dadurch gelöst werden, dass der Kerndraht aus der ersten spiralförmigen Verbindungseinrichtung des medizinischen Implantats und der zweiten spiralförmigen Verbindungseinrichtung der Einführhilfe herausgezogen wird, so dass die erste und zweite spiralförmige Verbindungseinrichtung nicht mehr relativ zueinander fixiert werden.

Der erste Betriebszustand der erfindungsgemäßen Vorrichtung entspricht dabei dem Zustand während des Transports des medizinischen Implantats zur Implantationsstelle, beispielsweise durch einen Katheter. Dementsprechend ist in dem zweiten Betriebszustand die Verbindung zwischen dem medizinischen Implantat und der Einführhilfe gelöst.

Das proximale Ende einer medizinischen Vorrichtung, wie beispielsweise des medizinischen Implantats, im Sinne der Erfindung ist das Ende der medizinischen Vorrichtung, das dem Anwender bzw. dem Arzt zugewandt angeordnet ist. Das distale Ende einer medizinischen Vorrichtung, beispielsweise der Einführhilfe, im Sinne der Erfindung ist das Ende der medizinischen Vorrichtung, welches von dem Anwender bzw. dem Arzt abgewandt angeordnet ist.

In einer Variante des erfindungsgemäßen Systems ist die Einführhilfe als Wendel ausgebildet, wobei die einzelnen Windungen der Wendel außerhalb der ersten spiralförmigen Verbindungseinrichtung vorzugsweise direkt benachbart zueinander angeordnet sind. Die Ausbildung der Einführhilfe als Wendel hat den Vorteil, dass die erste spiralförmige Verbindungseinrichtung auf einfache Art und Weise dadurch ausgebildet werden kann, dass der Abstand zwischen den Windungen der Wendel im Bereich der ersten spiralförmigen Verbindungseinrichtung auf einen vorbestimmten Abstand gebracht werden. Im Bereich außerhalb der ersten spiralförmigen Verbindungseinrichtung sind die Wendeln der Einführhilfe vorzugsweise direkt benachbart zueinander angeordnet, so dass die Einführhilfe zylinderförmig mit einer geschlossenen Zylinderoberfläche ausgebildet ist. Dadurch weist die Einführhilfe in Längsrichtung der Einführhilfe eine hohe Steifigkeit auf, ist jedoch gleichzeitig biegbar um beispielsweise einem gekrümmten Verlauf eines Katheters durch die Gefäße des menschlichen oder tierischen Körpers zu folgen.

Nach einer weiteren Variante des erfindungsgemäßen Systems ist das medizinische Implantat als Wendel ausgebildet, wobei die einzelnen Windungen der Wendel außerhalb der zweiten spiralförmigen Verbindungseinrichtung vorzugsweise zumindest teilweise benachbart zueinander angeordnet sind. Analog zu einer als Wendel ausgebildeten Einführhilfe lässt sich dadurch die zweite spiralförmige Verbindungseinrichtung auf einfache Art und Weise dadurch herstellen, dass die einzelnen Windungen der Wendel des medizinischen Implantats auf einen vorgegebenen Abstand gebracht werden. Im implantierten Zustand weist das medizinische Implantat häufig Krümmungen auf, so dass das als Wendel ausgebildete, medizinische Implantat in diesen Bereichen Wendeln aufweist, die zumindest im inneren Bereich benachbart zueinander angeordnet sind, gleichzeitig im äußeren Bereich jedoch leicht voneinander beabstandet sind.

In einer besonders zweckmäßigen Variante des erfindungsgemäßen Systems sind die einzelnen Windungen der ersten spiralförmigen Verbindungseinrichtung und oder der zweiten spiralförmigen Verbindungseinrichtung voneinander beabstandet. Dadurch können die erste spiralförmige Verbindungseinrichtung und die zweite spiralförmige Verbindungseinrichtung auf einfache Art und Weise ineinander verschachtelt werden und der Kerndraht durch die erste und die zweite spiralförmige Verbindungseinrichtung durchgeführt werden, zur Fixierung des ersten Betriebszustandes.

Nach einer besonders zweckmäßigen Variante des erfindungsgemäßen Systems entspricht der Abstand der einzelnen Windungen der ersten spiralförmigen Verbindungseinrichtung und/oder der zweiten spiralförmigen Verbindungseinrichtung dem 1,0- bis 2,5-fachen des Drahtdurchmessers der ersten spiralförmigen Verbindungseinrichtung und/oder der zweiten spiralförmigen Verbindungseinrichtung. Dadurch ist gewährleistet, dass die erste spiralförmige Verbindungseinrichtung von der zweiten spiralförmigen Verbindungseinrichtung nach Implantation des medizinischen Implantats und Entfernen des Kerndrahts gelöst werden kann.

Gemäß einer weiteren Variante des erfindungsgemäßen Systems besteht das medizinische Implantat, die Einführhilfe, die erste spiralförmige Verbindungseinrichtung, die zweite spiralförmige Verbindungseinrichtung und/oder der Kerndraht zumindest teilweise aus einem Formgedächtnismaterial, vorzugsweise aus einer Nickel-Titan-Legierung wie Nitinol.

Zweckmäßigerweise ist die erste spiralförmige Verbindungseinrichtung oder die zweite spiralförmige Verbindungseinrichtung aus einem Draht mit einem Durchmesser zwischen 0,3 mm und 1,5 mm hergestellt, vorzugsweise zwischen 0,5 mm und 1,0 mm und weiter bevorzugt zwischen 0,6 mm und 0,7 mm gebildet.

Nach einer bevorzugten Variante des erfindungsgemäßen Systems ist die erste spiralförmige Verbindungseinrichtung zu dem medizinischen Implantat abgewinkelt angeordnet. Zwischen den Windungen der ersten spiralförmigen Verbindungseinrichtung und dem medizinischen Implantat bildet sich durch die Abwinklung der ersten spiralförmigen Verbindungseinrichtung zu dem medizinischen Implantat eine Durchbrechung, durch welche der Kerndraht hindurchtreten kann. Somit ragt der Kerndraht in dem ersten Betriebszustand nicht in das medizinische Implantat, was einen negativen Einfluss auf die Entfaltung des medizinischen Implantats während der Implantation haben könnte.

Gemäß einer Variante der Erfindung ist die erste spiralförmige Verbindungseinrichtung oder die zweite spiralförmige Verbindungseinrichtung entlang der Längsachse des Kerndrahts gekrümmt ausgebildet, wobei die gekrümmte erste spiralförmige Verbindungseinrichtung in dem ersten Betriebszustand gegen elastische Rückstellkräfte mittels des Kerndrahts in einer gestreckten Lage entlang der Längsachse des Kerndrahts gehalten wird. Nach dem Entfernen des Kerndrahts nimmt die erste bzw. zweite spiralförmige Verbindungseinrichtung aufgrund der elastischen Rückstellkräfte ihre gekrümmte Ausrichtung ein, wodurch sich die erste spiralförmige Verbindungseinrichtung von der zweiten spiralförmigen Verbindungseinrichtung, oder umgekehrt, entfernt. Somit löst sich die Verbindung zwischen der ersten und der zweiten spiralförmigen Verbindungseinrichtung automatisch nach dem Entfernen des Kerndrahts.

Nach einer alternativen Variante des erfindungsgemäßen Systems sind die erste spiralförmige Verbindungseinrichtung und die zweite spiralförmige Verbindungseinrichtung entlang der Längsachse des Kerndrahts gekrümmt ausgebildet, wobei die Krümmung der ersten spiralförmigen Verbindungseinrichtung entgegengesetzt zu der Krümmung der zweiten spiralförmigen Verbindungseinrichtung ausgebildet ist, und wobei die gekrümmten ersten und zweiten spiralförmigen Verbindungseinrichtungen in dem ersten Betriebszustand gegen elastische Rückstellkräfte mittels des Kerndrahts in einer gestreckten Lage entlang der Längsachse des Kerndrahts gehalten werden. Nach dem Entfernen des Kerndrahts nehmen sowohl die erste spiralförmige Verbindungseinrichtung wie auch die zweite spiralförmige Verbindungseinrichtung aufgrund der elastischen Rückstellkräfte ihre gekrümmte Ausrichtung ein, so dass sich die erste spiralförmige Verbindungseinrichtung von der zweiten spiralförmigen Verbindungseinrichtung nach dem Entfernen des Kerndrahts löst.

Erfindungsgemäß ist die Einführhilfe zylinderförmig ausgebildet und weist einen Hohlraum entlang der Längsachse auf, wobei der Kerndraht innerhalb des Hohlraums angeordnet ist und relativ zu der Einführhilfe entlang der Längsachse der Einführhilfe bewegbar ist. Der Kerndraht ist somit innerhalb des Hohlraums der Einführhilfe bewegbar angeordnet und kann in dem ersten Betriebszustand durch die erste spiralförmige Verbindungseinrichtung des medizinischen Implantats und die zweite spiralförmige Verbindungseinrichtung der Einführhilfe geführt werden, sobald die erste spiralförmige Verbindungseinrichtung in die zweite spiralförmige Verbindungseinrichtung eingreift, so dass die Verbindung zwischen dem medizinischen Implantat und der Einführhilfe in dem ersten Betriebszustand gewährleistet ist.

Nach einer weiteren Variante der Erfindung weist der Kerndraht am distalen Ende eine Querschnittsvergrößerung auf, welche vorzugsweise größer ist als die Innendurchmesser der spiralförmigen Enden des medizinischen Implantats und der Einführhilfe. Dadurch wird ein unbeabsichtigtes Lösen des medizinischen Implantats von der Einführhilfe vermieden. Beispielsweise wird der Kerndraht in dem ersten Betriebszustand durch die Wandung des medizinischen Implantats nach außen geführt und nachfolgend wird der im wesentlichen runde Querschnitt durch eine Quetschung, auch Prägung genannt, derart verformt, dass der Querschnitt des Kerndrahtes größer ist als die Innendurchmesser der spiralförmigen Enden des medizinischen Implantats und der Einführhilfe. Beim Abwerfen des medizinischen Implantats wird der Kerndraht mit der Querschnittsvergrößerung durch die spiralförmigen Enden des medizinischen Implantats und der Einführhilfe gezogen.

Nachfolgend wird die Erfindung anhand der in den Figuren dargestellten Ausführungsbeispiele näher erläutert.

Es zeigen:
- Figur 1: Ein erfindungsgemäßes System zum Verbinden eines medizinischen Implantats mit einer Einführhilfe in einem ersten Betriebszustand,
- Figur 2: das System aus Figur 1 in einem zweiten Betriebszustand, und
- Figur 3: ein weiteres erfindungsgemäßes System zum Verbinden eines medizinischen Implantats mit einer Einführhilfe in einem ersten Betriebszustand.

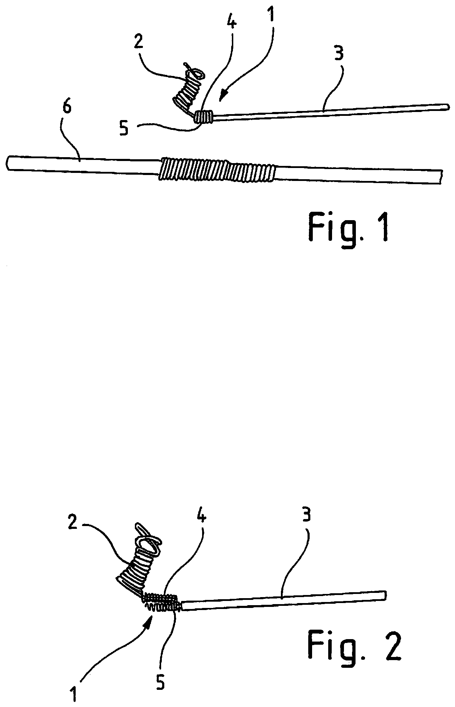

In Figur 1 ist ein erfindungsgemäßes System 1 zum Verbinden eines medizinischen Implantats 2 mit einer Einführhilfe 3 in einem ersten Betriebszustand dargestellt. Das in Figur 1 dargestellte System 1 umfasst eine erste spiralförmige Verbindungseinrichtung 4 am proximalen Ende des medizinischen Implantats 2, eine zweite spiralförmige Verbindungseinrichtung 5 am distalen Ende der Einführhilfe 3 und einen Kerndraht 6. In dem in Figur 1 dargestellten, ersten Betriebszustand des erfindungsgemäßen Systems 1 greifen die erste spiralförmige Verbindungseinrichtung 4 des medizinischen Implantats 2 und die zweite spiralförmige Verbindungseinrichtung 5 der Einführhilfe 3 zumindest teilweise ineinander und der Kerndraht 6 erstreckt sich durch die erste und zweite spiralförmige Verbindungseinrichtung 4, 5 hindurch, so dass der Kerndraht 6 in dem ersten Betriebszustand des Systems 1 die erste spiralförmige Verbindungseinrichtung 4 und die zweite spiralförmige Verbindungseinrichtung 5 relativ zueinander fixiert.

Die Einführhilfe 3 des erfindungsgemäßen Systems 1 aus Figur 1 ist als Wendel ausgebildet, wobei die einzelnen Windungen der Wendel außerhalb der ersten spiralförmigen Verbindungseinrichtung 4 direkt benachbart zueinander angeordnet sind. Das medizinische Implantat 2 des erfindungsgemäßen Systems 1 aus Figur 1 ist ebenfalls als Wendel ausgebildet, wobei die einzelnen Windungen der Wendel außerhalb der zweiten spiralförmigen Verbindungseinrichtung 5 zumindest teilweise benachbart zueinander angeordnet sind.

Wie Figur 1 entnommen werden kann, sind die einzelnen Windungen der ersten spiralförmigen Verbindungseinrichtung 4 und die einzelnen Windungen der zweiten spiralförmigen Verbindungseinrichtung 5 voneinander beabstandet. Der Abstand der einzelnen Windungen der ersten spiralförmigen Verbindungseinrichtung 4 und der Abstand der einzelnen Windungen der zweiten spiralförmigen Verbindungseinrichtung 5 entspricht dem 1,0 - 2,5-fachen des Drahtdurchmessers der ersten und zweiten spiralförmigen Verbindungseinrichtung 4,5 wobei die erste spiralförmige Verbindungseinrichtung 4 und/oder die zweite spiralförmige Verbindungseinrichtung 5 aus einem Draht mit einem Durchmesser zwischen 0,3 mm und 1,5 mm, vorzugsweise zwischen 0,5 mm und 1,0 mm und weiter bevorzugt zwischen 0,6 mm und 0,7 mm gebildet werden.

Zweckmäßigerweise bestehen das medizinische Implantat 2, die Einführhilfe 3, die erste spiralförmige Verbindungseinrichtung 4, die zweite spiralförmige Verbindungseinrichtung 5 und/oder der Kerndraht 6 zumindest teilweise aus einem Formgedächtnismaterial, vorzugsweise aus einer Nickel-Titan-Legierung wie Nitinol.

Wie der Figur 1 weiterhin entnommen werden kann, ist die erste spiralförmige Verbindungseinrichtung 4 zu dem medizinischen Implantat 2 abgewinkelt angeordnet. Dadurch kann der Kerndraht 6 durch die einzelnen Windungen der ersten spiralförmigen Verbindungseinrichtung 4 austreten, so dass sich der Kerndraht 6 nicht in das medizinische Implantat 2 erstreckt und dessen vorgegebene Form beeinflusst.

Die Einführhilfe 3 des erfindungsgemäßen Systems 1 aus Figur 1 ist zylinderförmig ausgebildet und weist einen Hohlraum entlang der Längsachse der Einführhilfe 3 auf. Der Kemdraht 6 des Systems 1 ist innerhalb des Hohlraums angeordnet und relativ zu der Einführhilfe 3 entlang der Längsachse der Einführhilfe 3 bewegbar.

In Figur 2 ist das erfindungsgemäße System 1 aus Figur 1 in einem zweiten Betriebszustand dargestellt. In dem zweiten Betriebszustand wurde der Kerndraht 6 innerhalb der Einführhilfe 3 zurückgezogen, wodurch die Verbindung zwischen der Einführhilfe 3 und dem medizinischen Implantat 2 gelöst wurde. Die erste spiralförmige Verbindungseinrichtung 4 des medizinischen Implantats 2 wurde nachfolgend von der zweiten spiralförmigen Verbindungseinrichtung 5 der Einführhilfe 3 getrennt, so dass die erste spiralförmige Verbindungseinrichtung 4 und die zweite spiralförmige Verbindungseinrichtung 5 nicht mehr ineinandergreifen. In dem dargestellten zweiten Betriebszustand befindet sich das medizinische Implantat 2 an dem gewünschten Implantationsort innerhalb des menschlichen oder tierischen Körpers. Nach dem Lösen der Verbindung zwischen dem medizinischen Implantat 2 und der Einführhilfe 3 kann die Einführhilfe 3 durch den zur Implantation verwendeten Katheter (nicht dargestellt) zurückgezogen werden.

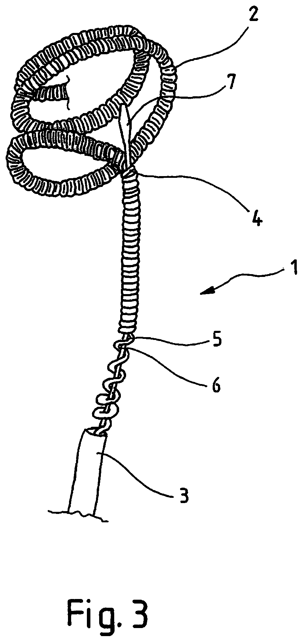

Das in Figur 3 dargestellte erfindungsgemäße System 1 zum Verbinden eines medizinischen Implantats 2 mit einer Einführhilfe 3 unterscheidet sich von dem System 1 aus Fig. 1 durch die Ausgestaltung des Kerndrahts. In dem Ausführungsbeispiel gemäß Figur 3 weist der Kerndraht 6 am distalen Ende eine Querschnittsvergrößerung 7 auf, welche größer ist als der Innendurchmesser der spiralförmigen Enden 4, 5 des medizinischen Implantats 2 und der Einführhilfe 3. Dadurch wird ein unbeabsichtigtes Lösen des medizinischen Implantats 2 von der Einführhilfe 3 vermieden. Der Kerndraht 6 ist in dem in Figur 3 dargestellten ersten Betriebszustand durch die Wandung des medizinischen Implantats 2 nach außen geführt. Nach dem Herausführen aus dem medizinischen Implantat 2 wurde der Kerndraht 6 gequetscht, zur Erzeugung der Querschnittvergrößerung 7.

### Bezugszeichenliste

- 1: System
- 2: Implantat
- 3: Einführhilfe
- 4: Erste spiralförmige Verbindungseinrichtung
- 5: Zweite spiralförmige Verbindungseinrichtung
- 6: Kerndraht
- 7: Querschnittsvergrößerung Kerndraht

## Patentansprüche

1. System (1) zum Verbinden eines medizinischen Implantats (2) mit einer Einführhilfe (3), umfassend:
eine erste spiralförmige Verbindungseinrichtung (4) am proximalen Ende des medizinischen Implantats (2),
eine zweite spiralförmige Verbindungseinrichtung (5) am distalen Ende der Einführhilfe (3), **gekennzeichnet durch** ein Kerndraht (6), welcher in einem ersten Betriebszustand des Systems (1) die erste spiralförmige Verbindungseinrichtung (4) und die zweite spiralförmige Verbindungseinrichtung (5) relativ zueinander fixiert,
wobei in dem ersten Betriebszustand die Wendeln der ersten spiralförmigen Verbindungseinrichtung (4) und die Wendeln der zweiten spiralförmigen Verbindungseinrichtung (5) zumindest teilweise ineinander greifen und sich der Kerndraht (6) durch die erste spiralförmige Verbindungseinrichtung (4) und die zweite spiralförmige Verbindungseinrichtung (5) hindurch erstreckt, und
wobei die Einführhilfe (3) zylinderförmig ausgebildet ist und einen Hohlraum entlang der Längsachse aufweist, und wobei der Kerndraht (6) innerhalb des Hohlraums angeordnet ist und relativ zur der Einführhilfe (3) entlang der Längsachse der Einführhilfe (3) bewegbar ist.

2. System (1) nach Anspruch 1,
wobei die Einführhilfe (3) als Wendel ausgebildet ist, wobei die einzelnen Windungen der Wendeln außerhalb der ersten spiralförmigen Verbindungseinrichtung (4) vorzugsweise direkt benachbart zueinander angeordnet sind.

3. System (1) nach Anspruch 1 oder 2,
wobei das medizinische Implantat (2) als Wendel ausgebildet ist, wobei die einzelnen Windungen der Wendel außerhalb der zweiten spiralförmigen Verbindungseinrichtung (5) vorzugsweise zumindest teilweise benachbart zueinander angeordnet sind.

4. System (1) nach einem der Ansprüche 1 bis 3,
wobei die einzelnen Windungen der ersten spiralförmigen Verbindungseinrichtung (4) und/oder der zweiten spiralförmigen Verbindungseinrichtung (5) voneinander beabstandet sind.

5. System (1) nach Anspruch 4,
wobei der Abstand der einzelnen Windungen der ersten spiralförmigen Verbindungseinrichtung (4) und/oder der zweiten spiralförmigen Verbindungseinrichtung (5) dem 1,0 bis 2,5-fachen des Drahtdurchmessers der ersten spiralförmigen Verbindungseinrichtung (4) und/oder der zweiten spiralförmigen Verbindungseinrichtung (5) entspricht.

6. System (1) nach einem der Ansprüche 1 bis 5,
wobei das medizinische Implantat (2), die Einführhilfe (3), die erste spiralförmige Verbindungseinrichtung (4), die zweite spiralförmige Verbindungseinrichtung (5) und/oder der Kerndraht (6) zumindest teilweise aus einem Formgedächtnismaterial besteht, vorzugsweise aus einer Nickel-Titan-Legierung wie Nitinol.

7. System (1) nach einem der Ansprüche 1 bis 6,
wobei die erste spiralförmige Verbindungseinrichtung (4) und/oder die zweite spiralförmige Verbindungseinrichtung (5) aus einem Draht mit einem Durchmesser zwischen 0,3 mm und 1,5 mm, vorzugsweise zwischen 0,5 mm und 1,0 mm und weiter bevorzugt zwischen 0,6 mm und 0,7 mm gebildet ist.

8. System (1) nach einem der Ansprüche 1 bis 7,
wobei die erste spiralförmige Verbindungseinrichtung (4) zu dem medizinischen Implantat (2) abgewinkelt angeordnet ist.

9. System (1) nach einem der Ansprüche 1 bis 8,
wobei die erste spiralförmige Verbindungseinrichtung (4) oder die zweite spiralförmige Verbindungseinrichtung (5) entlang der Längsachse des Kerndrahts (6) gekrümmt ausgebildet ist, wobei die gekrümmte erste spiralförmige Verbindungseinrichtung (4) oder die zweite spiralförmige Verbindungseinrichtung (5) in dem ersten Betriebszustand gegen elastische Rückstellkräfte mittels des Kerndrahts (6) in einer gestreckten Lage entlang der Längsachse des Kerndrahts (6) gehalten wird.

10. System (1) nach einem der Ansprüche 1 bis 9,
wobei die erste spiralförmige Verbindungseinrichtung (4) und die zweite spiralförmige Verbindungseinrichtung (5) entlang der Längsachse des Kerndrahts (6) gekrümmt ausgebildet sind, wobei die Krümmung der ersten spiralförmigen Verbindungseinrichtung (4) entgegengesetzt zur Krümmung der zweiten spiralförmigen Verbindungseinrichtung (5) ausgebildet ist, und wobei die gekrümmten ersten und zweiten spiralförmigen Verbindungseinrichtungen (4, 5) in dem ersten Betriebszustand gegen elastische Rückstellkräfte mittels des Kemdrahts (6) in einer gestreckten Lage entlang der Längsachse des Kerndrahts (6) gehalten werden.

11. System (1) nach einem der Ansprüche 1 bis 10, wobei der Kerndraht (6) am distalen Ende eine Querschnittsvergrößerung aufweist, welche vorzugsweise größer ist als die Innendurchmesser der spiralförmigen Enden des medizinischen Implantats und der Einführhilfe.

## Claims

1. System (1) for connecting a medical implant (2) to an insertion aid (3), comprising:
a first helical connecting member (4) at the proximal end of the medical implant (2),
a second helical connecting member (5) at the distal end of the insertion aid (3), **characterized by**
a core wire (6), which in a first operating state of the system (1) locks the first helical connecting member (4) and the second helical connecting member (5) relative to each other,
wherein in the first operating state the turns of the first helical connecting member (4) and the turns of the second helical connecting member (5) at least partially engage each other and the core wire (6) extends through the first helical connecting member (4) and the second helical connecting member (5), and
wherein the insertion aid (3) is cylindrical and has a hollow space along the longitudinal axis, and wherein the core wire (6) is located within the hollow space and movable relative to the insertion aid (3) along the longitudinal axis of the insertion aid (3).

2. System (1) according to claim 1,
wherein the insertion aid (3) is a spiral, wherein the single turns of the spiral outside the first helical connecting member (4) are preferably located directly adjacent to each other.

3. System (1) according to claim 1 or 2,
wherein the medical implant (2) is a spiral, wherein the single turns of the spiral outside the second helical connecting member (5) are preferably at least partly located adjacent to each other.

4. System (1) according to one of claims 1 to 3,
wherein the single turns of the first helical connecting member (4) and/or the second helical connecting member (5) are spaced from each other.

5. System (1) according to claim 4,
wherein the spacing of the single turns of the first helical connecting member (4) and/or the second helical connecting member (5) corresponds to 1,0 to 2,5 times the diameter of the wire of the first helical connecting member (4) and/or the second helical connecting member (5).

6. System (1) according to one of claims 1 to 5,
wherein the medical implant (2), the insertion aid (3), the first helical connecting member (4), the second helical connecting member (5) and/or the core wire (6) at least partly consist of a shape-memory material, preferably of a nickel-titanium alloy like nitinol.

7. System (1) according to one of claims 1 to 6,
wherein the first helical connecting member (4) and/or the second helical connecting member (5) are built of a wire with a diameter between 0,3 mm and 1,5 mm, preferably between 0,5 mm and 1,0 mm and further preferred between 0,6 mm and 0,7 mm.

8. System (1) according to one of claims 1 to 7,
wherein the first helical connecting member (4) is arranged angled to the medical implant (2).

9. System (1) according to one of claims 1 to 8,
wherein the first helical connecting member (4) or the second helical connecting member (5) is curved along the longitudinal axis of the core wire (6), wherein the curved first helical connecting member (4) or the second helical connecting member (5) in the first operating state is fixed against elastic restoring forces in an elongated state along the longitudinal axis of the core wire (6) by the core wire (6).

10. System (1) according to one of claims 1 to 9,
wherein the first helical connecting member (4) and the second helical member (5) are curved along the longitudinal axis of the core wire (6), wherein the curvature of the first helical connecting member (4) is opposite to the curvature of the second helical connecting member (5), and wherein the curved first and second helical connecting members (4, 5) in the first operating condition are fixed against elastic restoring forces in an elongated state along the longitudinal axis of the core wire (6) by the core wire (6).

11. System (1) according to one of claims 1 to 10,
wherein the core wire (6) has an enlarged cross-section at the distal end, which is preferably larger than the inner diameter of the helical ends of the medical implant and the insertion aid.

## Revendications

1. Système (1) destiné à relier un implant médical (2) à un dispositif facilitant l'insertion (3), comprenant :
un premier élément de liaison hélicoïdal (4) au niveau de l'extrémité proximale de l'implant médical (2),
un second élément de liaison hélicoïdal (5) au niveau de l'extrémité distale du dispositif facilitant l'insertion (3), **caractérisé par**
un fil central (6), qui, dans un premier état de fonctionnement du système (1), bloque le premier élément de liaison hélicoïdal (4) et le second élément de liaison hélicoïdal (5) l'un par rapport à l'autre,
dans lequel, dans le premier état de fonctionnement, les spires du premier élément de liaison hélicoïdal (4) et les spires du second élément de liaison hélicoïdal (5) viennent au moins partiellement en prise les unes avec les autres et le fil central (6) s'étend dans le premier élément de liaison hélicoïdal (4) et le second élément de liaison hélicoïdal (5), et
dans lequel le dispositif facilitant l'insertion (3) est cylindrique et possède un espace creux le long de l'axe longitudinal, et dans lequel le fil central (6) est situé à l'intérieur de l'espace creux et mobile par rapport au dispositif facilitant l'insertion (3) le long de l'axe longitudinal du dispositif facilitant l'insertion (3).

2. Système (1) selon la revendication 1,
dans lequel le dispositif facilitant l'insertion (3) est une spirale, les spires individuelles de la spirale à l'extérieur du premier élément de liaison hélicoïdal (4) étant de préférence situées de manière directement adjacente les unes aux autres.

3. Système (1) selon la revendication 1 ou 2,
dans lequel l'implant médical (2) est une spirale, les spires individuelles de la spirale à l'extérieur du second élément de liaison hélicoïdal (5) étant de préférence au moins partiellement situées de manière adjacente les unes aux autres.

4. Système (1) selon l'une quelconque des revendications 1 à 3,
dans lequel les spires individuelles du premier élément de liaison hélicoïdal (4) et/ou du second élément de liaison hélicoïdal (5) sont écartées les unes des autres.

5. Système (1) selon la revendication 4,
dans lequel l'écartement des spires individuelles du premier élément de liaison hélicoïdal (4) et/ou du second élément de liaison hélicoïdal (5) correspond 1,0 à 2,5 fois au diamètre du fil du premier élément de liaison hélicoïdal (4) et/ou du second élément de liaison hélicoïdal (5).

6. Système (1) selon l'une quelconque des revendications 1 à 5,
dans lequel l'implant médical (2), le dispositif facilitant l'insertion (3), le premier élément de liaison hélicoïdal (4), le second élément de liaison hélicoïdal (5) et/ou le fil central (6) consiste au moins en un matériau à mémoire de forme, de préférence en un alliage nickel-titane comme le nitinol.

7. Système (1) selon l'une quelconque des revendications 1 à 6,
dans lequel le premier élément de liaison hélicoïdal (4) et/ou le second élément de liaison hélicoïdal (5) sont constitués d'un fil ayant un diamètre compris entre 0,3 mm et 1,5 mm, de préférence entre 0,5 mm et 1,0 mm et de manière davantage préférée entre 0,6 mm et 0,7 mm.

8. Système (1) selon l'une quelconque des revendications 1 à 7,
dans lequel le premier élément de liaison hélicoïdal (4) est disposé de manière angulaire par rapport à l'implant médical (2).

9. Système (1) selon l'une quelconque des revendications 1 à 8,
dans lequel le premier élément de liaison hélicoïdal (4) ou le second élément de liaison hélicoïdal (5) est incurvé le long de l'axe longitudinal du fil central (6), dans lequel le premier élément de liaison hélicoïdal (4) incurvé ou le second élément de liaison hélicoïdal (5) dans le premier état de fonctionnement est fixé pour contrer des forces de rappel élastiques dans un état allongé le long de l'axe longitudinal du fil central (6) par le fil central (6).

10. Système (1) selon l'une quelconque des revendications 1 à 9,
dans lequel le premier élément de liaison hélicoïdal (4) et le second élément de liaison hélicoïdal (5) sont incurvés le long de l'axe longitudinal du fil central (6), dans lequel la courbure du premier élément de liaison hélicoïdal (4) est opposée à la courbure du second élément de liaison hélicoïdal (5), et dans lequel les premier et second éléments de liaison hélicoïdaux (4, 5) incurvés dans le premier état de fonctionnement sont fixés contre des forces de rappel élastiques dans un état allongé le long de l'axe longitudinal du fil central (6) par le fil central (6).

11. Système (1) selon l'une quelconque des revendications 1 à 10,
dans lequel le fil central (6) présente une coupe transversale élargie à l'extrémité distale, qui est de préférence plus large que le diamètre interne des extrémités hélicoïdales de l'implant médical et du dispositif facilitant l'insertion.
